**Europäisches Patentamt**

**European Patent Office**

**Office européen des brevets**

(11) Numéro de publication: **0 405 039**

**A1**

# (12) DEMANDE DE BREVET EUROPEEN

(21) Numéro de dépôt: **89402112.0**

(22) Date de dépôt: **25.07.89**

(51) Int. Cl.5: **A61M 5/32**

(30) Priorité: **27.06.89 FR 8908514**

(43) Date de publication de la demande:
**02.01.91 Bulletin 91/01**

(84) Etats contractants désignés:
**AT BE CH DE ES FR GB IT LI LU NL SE**

(71) Demandeur: **GUERINEAU POIRIER**
**228 quai de Stalingrad**
**F-92130 Issy-les-Moulineaux(FR)**

(72) Inventeur: **GUERINEAU POIRIER**
**228 quai de Stalingrad**
**F-92130 Issy-les-Moulineaux(FR)**

(54) **Combiné seringue-aiguille auto-escamotable.**

(57) La présente invention concerne les seringues.

La seringue selon l'invention comporte, en plus des éléments constitutifs habituels, un fourreau de protection (9) coulissant mû par un ressort (5) et dont le déclenchement automatique à la fin de l'injection permet de recouvrir définitivement l'aiguille, (3) rendant ladite seringue inutilisable et inoffensive.

FIG 2      FIG 3

**EP 0 405 039 A1**

La présente invention a pour objet une seringue comportant, outre les parties habituelles d'une seringue classique ( corps, aiguille, piston-poussoir), un fourreau de protection dans lequel l'ensemble solidaire seringue-aiguille coulisse librement, percé à une extrémité d'un trou permettant le passage de l'aiguille, et comprimant un ressort qui, libéré par le poussoir en fin d'injection, l'amènera à recouvrir totalement l'aiguille. En outre, une rondelle percée d'un trou trés fin et d'un diamètre convenable empêche l'aiguille de ressortir du fourreau aprés usage.

D'autres caractèristiques et avantages de la présente invention apparaîtront au cours de la description suivante donnée en regard des dessins annexés à titre illustratif mais nullement limitatif, dans lesquels:

- La figure 1 représente la seringue selon l'invention en vue générale et munie de son capuchon d'aiguille.

- La figure 2 représente la même seringue vue en coupe longitudinale avant usage.

- La figure 3 représente la seringue aprés usage vue en coupe longitudinale.

- La figure 4 représente le fourreau protecteur vu en coupe transversale selon le pointillé représenté sur la figure 1 ,avant la mise en place du combiné seringue-aiguille et sterilisation.

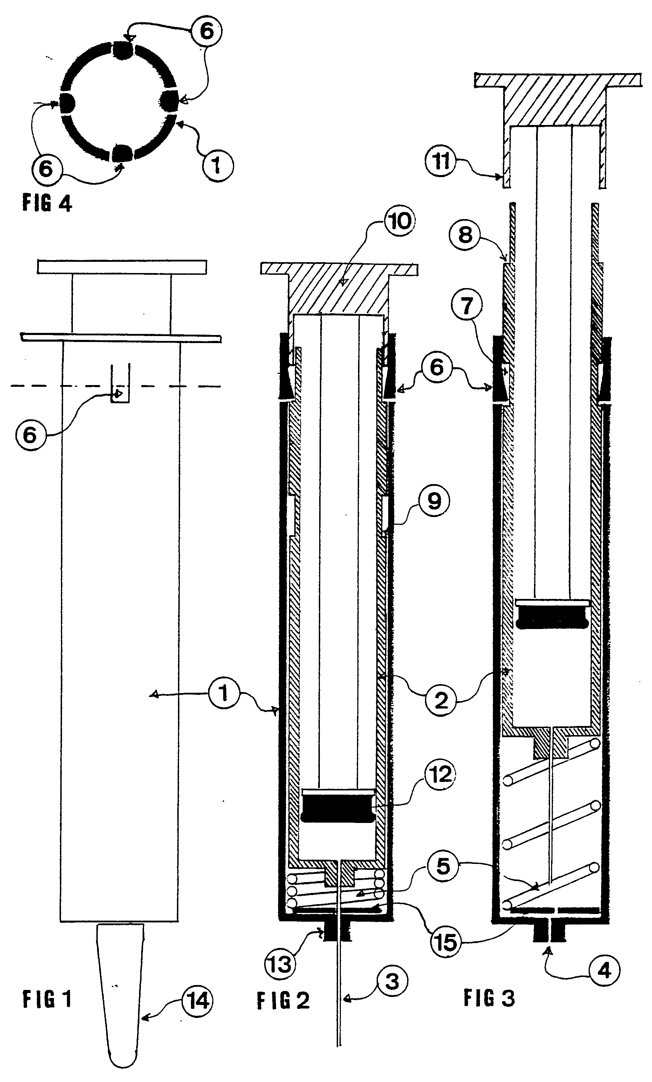

Revenons plus particulièrement aux figures 2,3 et 4

La seringue proprement dite 2 et son aiguille 3 sont placées dans le fourreau protecteur 1 en comprimant un ressort 5 ,métallique ou non, l'aiguille sortant du fourreau par le trou 4, en passant à travers la rondelle 15. Le corps de la seringue 2 comporte deux épaulements circulaires 8 et 9 distants d'une longueur légèrement supérieure à celle de l'aiguille. Ce corps, cylindrique, présente donc alternativement deux diamètres différends, ou plutôt, la paroi de la seringue est plus mince sur deux sections de sa longueur, à savoir:

- Depuis l'ouverture supérieure (extrémité opposée à l'aiguille), jusqu'à l'épaulement 8 formé d'une bague circulaire.

- Depuis la partie inférieure de cette bague jusqu'à l'épaulement 9. Cette section , 7 sur la figure 3, sera juste de la longueur des ergots, de manière que la bague 8 reste engagée dans le fourreau aprés la phase finale, interdisant ainsi l'introduction d'un corps étranger -ticket de metro par exemple- en vue d'écarter lesdits ergots afin de pouvoir réutiliser la seringue.

L'ensemble aiguille-seringue est maintenu au fond du fourreau par un certain nombre d' ergots 6 solidaires de celui-ci, et prenant appui sur le premier épaulement 8 de la seringue. Ces ergots, réalisés en matière plastique souple, sont moulés d'une pièce avec le fourreau, qui n'étant pas en contact avec le liquide à injecter, n'a pas à répondre à des critères particuliers de qualité dite alimentaire. .

La figure 4 montre la disposition de ces ergots. On voit que leur forme arrondie vers l'intérieur du fourreau permet, à la fabrication, le dégagement du poinçon de moulage après rotation. On se gardera, lors du montage avant stérilisation, d'enfoncer à fond le piston étanche 12 au fond de la seringue mais on laissera un espace de quelques millimètres entre le piston et le fond du réservoir comme le montre la figure 2.

Le remplissage aura lieu de manière classique par aspiration à travers l'aiguille en tirant le bouton poussoir 10, et l'injection pourra être effectuée après avoir chassé la bulle d'air. Arrivé en bout de course, le bouton poussoir 10 du piston engagera sa bague inférieure 11 autour de la partie la plus mince de la seringue, jusqu'à venir buter sur l'épaulement 8, chassant ainsi les ergots 6 vers l'extérieur, lorsque la pression du doigt sur le poussoir se relâchera, le ressort 5 ainsi libéré chassera la seringue dans le fourreau jusqu'à ce que le second épaulement 9 se présente en face des ergots qui, s'appuyant dessus, stopperont le mouvement. A ce moment, l'aiguille sera entièrement rentrée dans le fourreau et l'ensemble aiguille-seringue restera prisonnier dudit fourreau.

Le trou 4 étant percé au plus juste pour laisser passer l'aiguille 3 et celle-ci, la plus fine possible,étant toujours légèrement faussée lors de la pénétration dans la peau, il est virtuellement impossible de la faire ressortir du fourreau en appuyant sur le poussoir. De plus, la rondelle 15, réalisée en métal ou dans une matière plastique appropriée, étant d'un diamètre inférieur à celui du fourreau, sera libérée de l'aiguille et son trou central ne coincidera plus avec celui du fourreau, interdisant ainsi le passage de l'aiguille, même si l'on tente d'agrandir le trou 14 à l'aide d'un poinçon.

Il reste possible, si le diamètre de l'ensemble est trop important et constitue une gêne pour effectuer une autoinjection intraveineuse, de décentrer l'aiguille 3 au plus prés de la paroi cylindrique de la seringue 2 comme cela se pratique couramment avec les seringues ordinaires.

Le fourreau de protection 1 , réalisé dans un matériau suffisemment transparent pour permettre la lecture des graduations de la seringue, est muni, autour du trou 4 d'une partie cylindrique 13 recevant le capuchon protège aiguille 14.

## Revendications

1-Seringue caractèrisée en ce qu'elle comporte un corps 2 solidaire de l'aiguille creuse 3 et coulisse librement dans un fourreau protecteur 1, afin que, mûe par le ressort 5, elle vienne s'escamoter de toute la longuer de l'aiguille dans ledit fourreau.

2-Seringue selon la revendication 1 caractèrisée par le fait que le corps 2 comporte deux diamètres différends répartis en quatre sections:
-faible de l'ouverture supérieure à l'épaulement 8
-fort sur la longueur de cette bague
-faible entre la la bague 8 et l'épaulement 9 (section 7)
-fort de 9 à l'extrémité portant aiguille.

3-Seringue selon les revendication 1 et 2 caractèrisée par le fait que le corps 2 est maintenu en place par des ergots 6 de plastique souple solidaires du fourreau et prenant appui sur l'épaulement 8 dudit corps.

4-Seringue selon les revendications 1 et 2, caractèrisée par le fait qu'à la fin de l'injection, la bague 11 vient coiffer le haut du corps 2 jusqu'à l'épaulement 8 en chassant les ergots 6 vers l'extérieur, libérant ainsi l'action du ressort 5.

5-Seringue selon les revendications 1 à 3 caractèrisée par le faitqu'une fois libéreé de l'épaulement 8 etaprés une course calculée en fonction de la longueur de l'aiguille, le corps de la seringue se trouve de nouveau bloqué par l'action des ergots 6 sur l'épaulement 9, emprisonnant ladite aiguille dans le fourreau.

6-Seringue selon la revendication 1 caractèrisée par le fait que le ressort 5 peut être constitué de métal ou de tout

7-Seringue suivant la renvendication 3 caractèrisée par fait que les ergots souples 6 sont moulés d'un seul tenant avec le fourreau 1.

8-seringue selon la revendication 1 caractèrisée par le fait que le fourreau 1 comporte un trou pour l'aiguille ajusté au plus fin dans une saillie cylindrique 13 percée, portant ou non filetage extérieur,destinée à recevoir le capuchon cache aiguille 14.

9-Seringue selon les revendications 1 et 8 caractèrisée par le fait qu'une rondelle 15, percée en son centre au diamètre de l'aiguille et d'un diamètre inférieur à celui du fourreau, se trouvant libérée lorsque l'aiguille est chassée vers le haut, rend impossible un nouveau passage de celle-ci à travers le trou 4, même agrandi au moyen d'un outil pointu.

EP 0 405 039 A1

FIG 4

FIG 1

FIG 2

FIG 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 307 367  (AR. MA. S.R.L.) <br> * Figures 7-11; colonne 5, ligne 63 - colonne 7, ligne 27 * | 1,3,5,6 ,7 | A 61 M  5/32 |
| Y | | 9 | |
| A | | 4,8 | |
| | --- | | |
| Y | EP-A-0 276 160  (E.R. SQUIBB & SONS, INC.) <br> * Figures 3A-4B,14A,14B; colonne 6, ligne 52 - colonne 7, ligne 23; colonne 8, lignes 49-54 * | 9 | |
| | --- | | |
| A | EP-A-0 288 003  (HABLEY MEDICAL TECHNOLOGY CORP.) <br> * Figures 2-10; colonne 4, lignes 16-35; colonne 5, ligne 45 - colonne 7, ligne 43 * | 1,3,4,6 ,7 | |
| | --- | | |
| A | US-A-4 666 435  (BRAGINETZ) <br> * Figures 1,2; colonne 2, lignes 60-64; colonne 5, lignes 29,30 * <br> ----- | 8 | DOMAINES TECHNIQUES RECHERCHES (Int. Cl.5) <br><br> A 61 M |

Le présent rapport a été établi pour toutes les revendications

| Lieu de la recherche | Date d'achèvement de la recherche | Examinateur |
|---|---|---|
| LA HAYE | 15-02-1990 | SEDY, R. |

EPO FORM 1503 03.82 (P0402)